# EUROPEAN PATENT SPECIFICATION

(11) **EP 4 547 173 B1**
(45) Date of publication and mention of the grant of the patent: **09.12.2026**
(21) Application number: 22768637.5
(22) Date of filing: 17.08.2022
(51) Int. Cl.: A61F 9/008

(54) **HIGH-SPEED DEVICE FOR RE-SHAPING THE CORNEA**
HOCHGESCHWINDIGKEITSVORRICHTUNG ZUR UMFORMUNG DER HORNHAUT
DISPOSITIF À GRANDE VITESSE POUR LE REMODELAGE DE LA CORNÉE

(43) Date of publication of application: 07.05.2025
(73) Proprietor: Pflaum, Christian, 82347 Bernried (DE)
(72) Inventor: Pflaum, Christian, 82347 Bernried (DE)
(74) Representative: Vossius & Partner Patentanwälte Rechtsanwälte mbB
(86) International application number: PCT/EP2022/072929
(87) International publication number: WO 2024/037714

(56) References cited:
- WO-A1-2022/033701
- WO-A2-2012/167260
- US-A1- 2018 110 651
- US-B1- 6 508 812

## Description

The present invention relates to a high-speed device for re-shaping the cornea of a patient. Re-shaping the cornea of a patient has become a standard intervention during the last decades for various impairments. Usually, the surgeon creates a corneal flap, i.e., a thin slice of the cornea, utilizing a blade or a femtosecond laser. Once the flap has been cut and flipped aside, an excimer laser is used to remove tissue from the center of the cornea to re-shape it, thus correcting the patient's refractive error. Afterwards, the corneal flap is replaced and allowed to heal naturally.

While cutting the corneal flap with a femtosecond laser and/or re-shaping the cornea with an excimer laser, the patient's eye usually has to be fixated in a predefined position in order to avoid any cutting errors. Since the procedure typically takes tens of seconds, this process may be rather unpleasant for the patient. Prior art can be found in US 6508812 B1, WO 2012/167260 A2, US 2018/110651 A1 and WO 2022/033701 A1.

It is therefore an object of the present invention to provide an improved device for re-shaping the cornea of a patient which allows for cutting the corneal flap and/or re-shaping the cornea more quickly.

This object is achieved with a device according to claim 1. Preferred embodiments of the inventive device are described in the dependent claims.

Accordingly, the present invention relates to a device for re-shaping the cornea of the patient. The device comprises a laser source, a digital micromirror device (DMD) adapted to emit a pattern of multiple laser beams, a mounting mechanism for fixating the head of a patient, focusing optics adapted for focusing the pattern of multiple laser beams emitted by the DMD onto the cornea of the patient, an image sensor adapted to image the cornea of the patient, and a controller adapted to control, based on the image detected by the image sensor, the laser source and the DMD so as to generate one or more illumination patterns on the cornea of the patient which allow(s) for re-shaping the cornea of the patient in accordance with a predetermined target shape of the cornea of the patient.

The present invention is thus, inter alia, based on the idea to utilize a DMD for simultaneously interfering with millions of regions ("pixels") of the patient's cornea in order to cut and/or ablate these regions simultaneously. This allows for substantially reusing the total amount of time required for the procedure as prior art devices are based on scanning techniques which require a longer time until the entire region of interest has been reached by the laser.

Alternatively, the present invention may utilize a ferroelectric spatial light modulator (SLM) or a liquid crystal SLM having a repetition rate of at least 200 Hz, preferably at least 500 Hz instead of the DMD. Ferroelectric SLMs allow for switching on and off with frequencies of up to 5 kHz and may thus replace the DMD of the present invention. Accordingly, the present invention further relates to a device for re-shaping the cornea of the patient. The device comprises a laser source, a ferroelectric SLM (or a liquid crystal SLM having a repetition rate of at least 200 Hz, preferably at least 500 Hz) adapted to emit a pattern of multiple laser beams, a mounting mechanism for fixating the head of a patient, focusing optics adapted for focusing the pattern of multiple laser beams emitted by the ferroelectric SLM onto the cornea of the patient, an image sensor adapted to image the cornea of the patient, and a controller adapted to control, based on the image detected by the image sensor, the laser source and the ferroelectric SLM so as to generate one or more illumination patterns on the cornea of the patient which allow(s) for re-shaping the cornea of the patient in accordance with a predetermined target shape of the cornea of the patient. All features discussed herein in the context with the embodiment comprising the DMD may be analogously employed in an embodiment comprising a ferroelectric SLM.

Preferably, the laser source comprises one or more of a picosecond laser, a femtosecond laser and an excimer laser. Most preferably, a combination of a femtosecond laser and an excimer laser are being used.

Preferably, the device further comprises a prism or semi-transparent mirror positioned between the laser source and the DMD. This allows for a particularly compact setup.

The parameters of the laser source, the optics and the DMD are preferably chosen such that each of the multiple laser beams emitted by the DMD is adapted to evaporate a section of the cornea of the patient. For this purpose, it is preferred that each of the multiple laser beams has a fluence of at least 10 mJ/cm², preferably of at least 30 mJ/cm². Preferably, a fluence of 100-1000 mJ/cm² is used for a femtosecond laser and a fluence of 10-500 mJ/cm² for an excimer laser.

Preferably, the focusing optics comprises a lens having a numerical aperture of at least 0.5, more preferably of at least 0.8.

The device preferably further comprises a beam shaping device such as a flat top beam shaper, a matrix of laser zone plates or a spatial light modulator.

Preferably, the laser beam passes, in that order, through a prism or semi-transparent mirror, hits the digital micromirror device, and again passes through the prism or semi-transparent mirror before selectively illuminating the cornea. Utilizing such a prism or semi-transparent mirror is particularly advantageous in that this allows for a compact arrangement of the various components of the device used. The laser beam preferably further passes twice through a λ/4 plate positioned between the prism or the semi-transparent mirror and the digital micromirror device. Changing the polarization of the laser beam by means of such a λ/4 plate allows for directing the laser beam from the laser via the prism or semi-transparent mirror to the digital micromirror device, and again back through the prism or semi-transparent mirror before selectively illuminating a plurality of regions of the cornea.

The DMD comprises an array or a matrix of micromirrors which allow to selectively illuminate predetermined pixels on the cornea by adjusting respective micromirrors of the array or matrix. Thus, a huge number of pixels on the cornea may be illuminated simultaneously and in a well-controlled manner, which can be easily automatized. Depending on the number of micromirrors present in the DMD, millions of selected regions (i.e. pixels) of the cornea can be manipulated simultaneously during a timespan which is sufficient to ablate one selected region. Such digital micromirror devices are readily available and can be simply implemented into devices of the prior art.

It is further preferred to utilize a beam shaping device to create certain beam shapes that are advantageous for data recording. For example, a matrix of laser zone plates may be transmitted by the multiple laser beams originating from the DMD. These laser zone plates may, for example, be adapted to create a needle-like Bessel beam for each of the multiple laser beams.

A Bessel beam has the advantage of a substantially increased depth of focus. While the focus length of a regular Gaussian beam is in the order of the wavelength of the focused light, the focus length which can be achieved with a Bessel beam amounts to at least 4 times the wavelength of the focus light. At the same time, the width of the focus is about one half of the focus width which can be achieved by a Gaussian beam. Utilizing a Bessel beam shape also allows for ablating tissue of the cornea over an enhance fraction of the surface of the cornea without adjusting the focal depth as the relevant diameter of such Bessel beam will remain within acceptable ranges over a rather large vertical displacement.

Such Bessel beams may also be generated by means of other beam shaping devices. One particularly preferred example of a beam shaping device is a spatial light modulator, which is particularly versatile because it can be utilized to create Bessel beams, to allow for optical proximity control and to provide a phase-shift mask.

Preferably, the device further comprises one or more scanning devices for steering the pattern of multiple laser beams emitted by the DMD over the cornea of the patient. If the area of the cornea to be re-shaped during the intervention cannot be accessed in one shot via the DMD, the area accessible by the DMD may be shifted along the x- and y-directions utilizing such scanning devices, like polygon scanner, galvo scanner or acousto-optic deflector (AOD). Thus, once an array or matrix of pixels has been re-shaped, an adjacent array or matrix of pixels may be re-shaped by simply steering the pattern of multiple laser beams emitted by the DMD to an adjacent area. Preferably, the one or more scanning devices comprise one or a combination of one or more galvo scanners, one or more polygon scanners, and one or more acousto-optic deflectors (AOD). It is particularly preferred to use a combination of one galvo scanner and one polygon scanner and the polygon scanner will allow for scanning along a first fast axis, whereas the galvo scanner may be used for scanning along a second, preferably perpendicular, slow axis.

Preferably, the one or more scanning devices are arranged between the DMD and the focusing optics.

It is further preferred that the controller is adapted to control, based on the image detected by the image sensor, the laser source, the one or more scanning devices and the DMD so as to generate several illumination patterns on the cornea of the patient which allow for re-shaping the cornea of the patient in accordance with a predetermined target shape of the cornea of the patient.

As mentioned previously, the device according to the present invention may not only be used for re-shaping the cornea, but also for cutting the flap during a typical intervention. Thus, the controller is preferably adapted to control, based on the image detected by the image sensor, the laser source, and the DMD, optionally also the one or more scanning devices, so as to generate one or more illumination patterns on the cornea of the patient which allow(s) for creating a flap of the cornea of the patient in accordance with a predetermined shape of the flap of the cornea of the patient. Preferably, the laser source comprises a femtosecond laser and an excimer laser, wherein the controller is adapted to utilize the femtosecond laser for creating the flap and to utilize the excimer laser for re-shaping the cornea.

Since the device comprises, as discussed above, an image sensor, a single optical ("writing and reading") head may be utilized for both re-shaping the cornea ("writing") and imaging the re-shaped cornea ("reading"). Utilizing the prism or semi-transparent mirror discussed above allows for designing such combined writing and reading head in a particularly compact shape.

The device preferably further comprises a beam splitter between the prism or the semi-transparent mirror and the focusing optics for allowing light emitted from the cornea to pass to the image sensor. The device preferably further comprises a further light source (e.g. an LED) adapted to illuminate the cornea via the prism or semi-transparent mirror and the DMD during reading / imaging. Preferably, the light source emits linearly polarized light.

The image sensor may comprise a digital camera or other optical detector. Preferably, the image sensor comprises a single optical sensor with each "pixel" on the cornea being addressed by means of the DMD which allows for illuminating each "pixel" at a time. In an alternative reading mode, utilizing SIM, a certain illumination pattern ("structured illumination") may be generated by the DMD. In that case, the image sensor should comprise a digital camera or other multi-pixel detector. In a further reading mode, plane illumination may be provided by simply setting all micromirrors of the DMD to be "ON". Also in that case, the image sensor should comprise a digital camera or other multi-pixel detector.

Preferred embodiments of the present invention will be further elucidated with reference to the figures, which show:
- Fig. 1a: a schematic view of a device for re-shaping the cornea according to a preferred embodiment;
- Fig. 1b: schematic front, back, top and side views of a mounting mechanism for fixating a patient's head;
- Fig. 2: a schematic illustration of the generation of a Bessel beam;
- Fig. 3: a schematic illustration of the generation of a Bessel beam arranged in a matrix;
- Fig. 4: a schematic view of a section of a human eye illustrating flap removal;
- Fig. 5: a schematic view of a device for re-shaping the cornea according to another preferred embodiment;
- Fig. 6: a schematic view of a device for re-shaping the cornea according to another preferred embodiment;
- Fig. 7: a schematic view of a device for re-shaping the cornea according to another preferred embodiment;
- Fig. 8: a schematic view of a device for re-shaping the cornea according to another preferred embodiment; and
- Fig. 9: a schematic illustration of the scanning process.

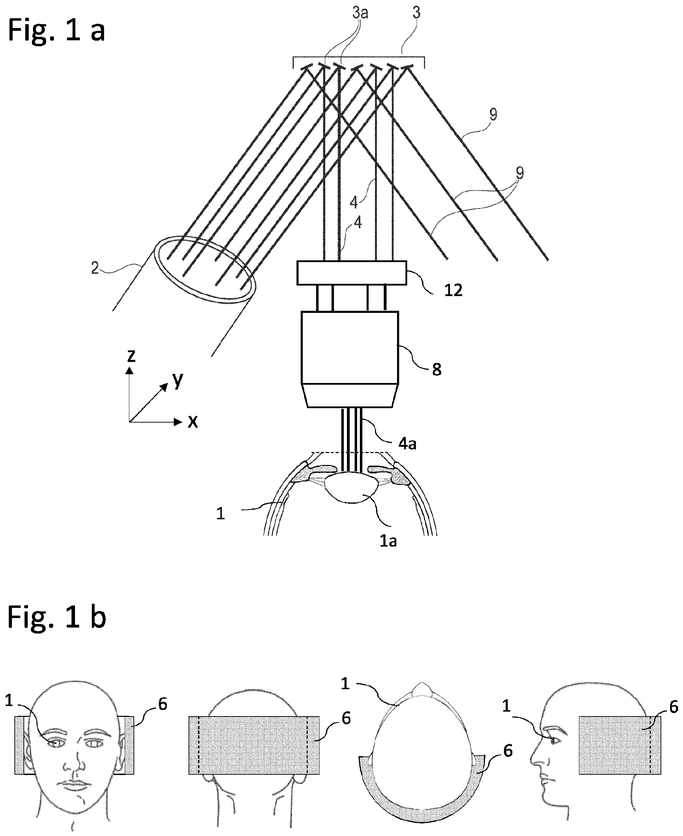

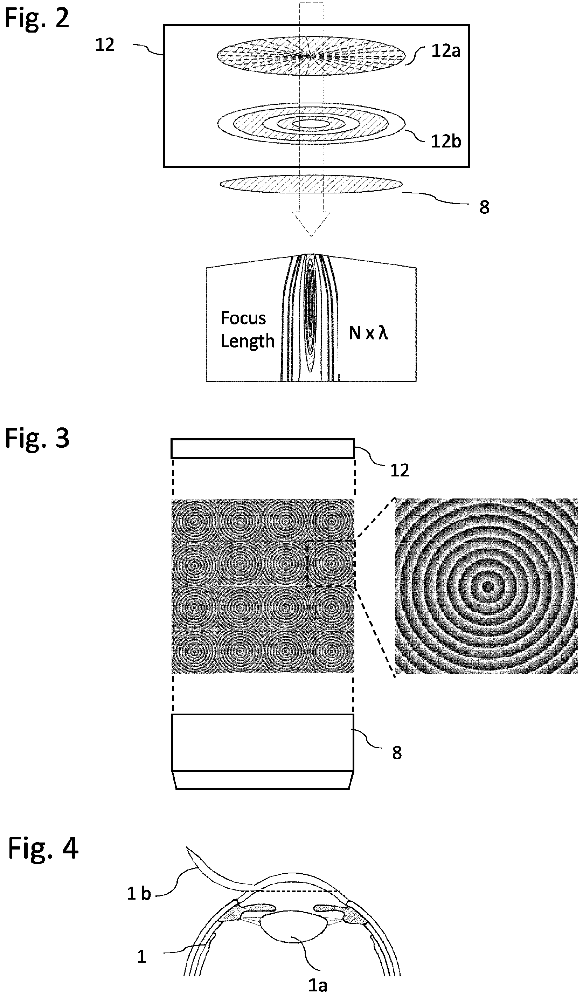

Fig. 1 shows a schematic illustration of a device for re-shaping the cornea of a patient according to a preferred embodiment of the present invention. The device comprises a laser source 2 emitting laser light onto a DMD 3 comprising multiple micromirrors 3a arranged in an array. The DMD 3 is adapted to emit multiple laser beams 4 along either a first direction (i.e., for re-shaping) or along a second direction (indicated with reference numeral 9) for each micromirror being in an "off" state diverting those laser beams 9 into a beam dump (not shown). Usually, the device will further comprise collimating optics (not shown in Fig. 1) for collimating laser light emitted by the laser source 2 onto the DMD 3. The device further comprises a mounting mechanism 6 (not shown in Fig. 1) for fixating the head of a patient, which is schematically shown in Fig. 2, and focusing optics 8 adapted for focusing each of the multiple laser beams 4 emitted by the DMD onto a thus fixated cornea. The focusing optics 8 may, for example, comprise standard microscope optics having a high numerical aperture.

As discussed previously, the device preferably comprises a beam shaping device to achieve, e.g., Bessel beams. For example, a matrix of laser zone plates 12 may be provided between the DMD 3 and the focusing optics 8 so as to shape each of the laser beams 4 into a Bessel beam shape. Each Bessel beam is then focussed onto the cornea by means of an attributed lens (e.g. Fresnel lens). This principle is further elucidated in Fig. 2 which shows (for a single beamlet) how a Bessel beam is generated by a combination of an optical element 12a creating circularly polarized light and a binary phase element 12b for creating a Bessel beam which is then focused onto the cornea by means of an attributed high NA lens 8 (or a Fresnel lens 8). As indicated also in Fig. 2, a focus length of N times the wavelength of the laser light may be achieved by using such a Bessel beam. Moreover, the focus has a much more cylindrical shape than a Gaussian beam.

Fig. 3 illustrates an alternative approach for creating a Bessel beam matrix utilizing a spatial light modulator as a phase-shift mask.

Fig. 4 shows a schematic view of a section of a human eye 1 illustrating a flap removal with the flap 1b being illustrated twice, once in its original arrangement and once after having been cut and flipped away. Reference numeral 1a refers to the lens.

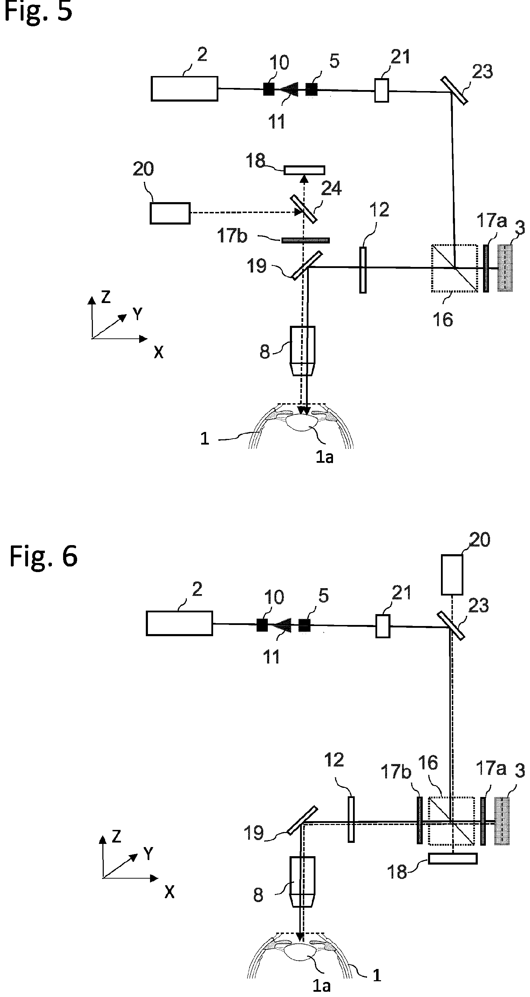

More details of another preferred embodiment of the inventive device are shown in Fig. 5. For example, Fig. 5 shows the collimating optics 5 for collimating laser light emitted by the laser source 2 onto the DMD 3 as well as further optical components such as an actuator 10 and a polarizer 11. Moreover, Fig. 5 further shows an optional flat top beam shaper 21 arranged between the collimating optics 5 and the DMD 3. More importantly, the preferred embodiment shown in Fig. 5 comprises a prism 16. The prism 16 (which could also be replaced by a semi-transparent mirror) is positioned between the laser source 2 and the DMD 3 in such a manner that light emitted from the laser source 2, in that order, passes through the prism 16, a λ/4 plate 17a, hits the DMD 3, and again passes through the λ/4 plate 17a and the prism 16 before selectively illuminating a plurality of regions of the cornea.

Linearly polarized light impinging on the prism 16 from the top light path will be reflected to the right side, i.e. towards the DMD 3. By passing through the λ/4 plate 17a twice the polarization axis of the laser light is, in sum, rotated by 90°. Thus, the again linearly polarized light impinging on the prism 16 from the right light path will pass through the prism 16.

A further λ/4 plate 17b may be provided (see, e.g., Fig. 6) to convert the linearly polarized light into circularly polarized light which is particularly advantageous for creating Bessel beams with the laser zone plate 12. Of course, if the laser zone plate 12 already comprises optical element 12a discussed above with respect to Fig. 2 the presence of an additional λ/4 plate 17b is not required (see Fig. 5).

The preferred embodiment shown in Fig. 5 further comprises an image sensor 18 configured to image the re-shaped cornea as well as a further light source 20 (e.g. an LED) adapted to illuminate the cornea. The linearly polarized light emitted from the light source 20 will be reflected at an additional beam splitter 24 towards the cornea. By passing through the λ/4 plate 17b twice the polarization axis of the laser light originating from the light source 20 is, in sum, rotated by 90° and can, thus, pass towards the image sensor 18. A beam splitter 19 between the prism 16 and the focusing optics 8, which reflects the wavelength of the laser light emitted from the laser source 2 and lets the wavelength(s) emitted by the further light source 20 pass, allows only light emitted from the cornea to pass the beam splitter 19 to the image sensor 18 and, thus, reduces any noise potentially caused by the ablation light.

The further light source 20 (e.g. an LED) may also be adapted to illuminate the cornea via the prism 16 and the DMD 3 during imaging as shown in Fig. 6. For this purpose, a further beam splitter 23 is provided, which reflects the wavelength of the laser light emitted from the laser source 2 and lets the wavelength(s) emitted by the further light source 20 pass. By passing through the λ/4 plate 17a twice the polarization axis of the laser light originating from the light source 20 is, again, rotated by 90°. Thus, the again linearly polarized light impinging on the prism 16 will pass through said prism 16 towards the cornea. Moreover, in this example, the image sensor 18, rather than being arranged on the optical axis of the focussing optics 8 as shown in Fig. 5, may also be arranged on a fourth side of the prism 16 as shown in Fig. 6. In this case, the beam splitter 19 would no longer be required and can be replaced by a mirror which is always reflecting. The λ/4 plates 17a and 17b again ensure that the laser light originating from the light source 20 is, in sum, rotated by 180°. Thus, the again linearly polarized light impinging on the prism 16 will be reflected towards the image sensor 18.

In reading/imaging mode, the DMD 3 may be utilized in different ways to illuminate the cornea with light emitted by the further light source 20. As discussed above, the cornea may be illuminated pixel by pixel using the DMD 3. In this case only a single detector is required in the image sensor and scanning of the image is performed by the DMD 3.

In an alternative reading mode, utilizing SIM, a certain illumination pattern ("structured illumination") will be generated by the DMD 3. In that case, the image sensor 18 should comprise a digital camera or other multi-pixel detector.

In a further reading mode, plane illumination may be provided by simply setting all micromirrors of the DMD 3 to be "ON". Also in that case, the image sensor 18 should comprise a digital camera or other multi-pixel detector.

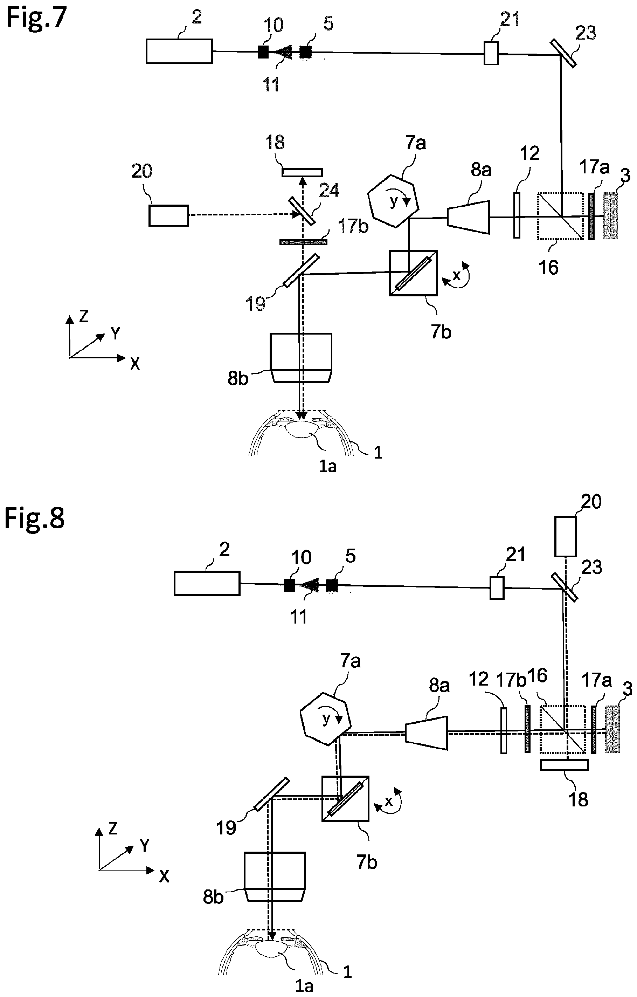

Of course, if the functionality of the DMD 3 is not used (plane illumination), the reading light path need not incorporate the DMD 3. Rather, the cornea may be illuminated with a further light source positioned, e.g., as shown in Fig. 7. Here, linearly polarized light emitted by the light source 20 is reflected by the beam splitter 24 towards the cornea. Since the light is circularly polarized after passing the λ/4 plate 17b said light will pass the beam splitter 19 and impinge on the cornea. The light reflected or otherwise emitted from the cornea again passes the beam splitter 19 and the λ/4 plate 17b. Subsequently, the light is again linearly polarized (rotated by 90°) and thus passes through the beam splitter 24 to the image sensor 18.

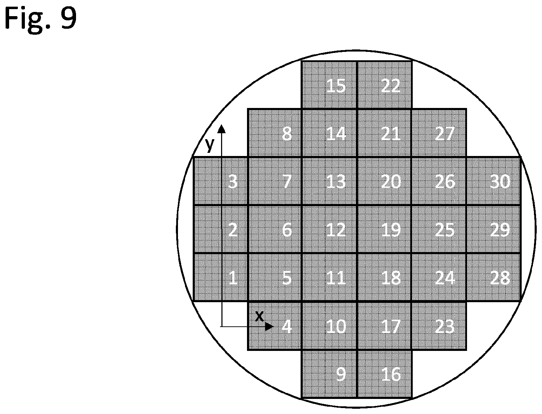

As mentioned previously, the device may further comprise one or more scanning devices for steering the pattern of multiple laser beams emitted by the DMD over the relevant surface area of the cornea of the patient. In the preferred embodiment shown in Fig. 7, a combination of one galvo scanner (7b) and one polygon scanner (7a) is used. The use of two scanners allows for scanning along two, preferably perpendicular, axes such as an x-axis and a y-axis (see Fig. 9). Scanning may then be performed along a first axis, e.g., the y-axis, and after one line along this first axis is completed, scanning is performed along the second axis, e.g., the x-axis. As will be evident from the scheme shown in Fig. 9, it may be beneficial to utilize a fast scanning process for the y-axis, whereas scanning along the x-axis may be performed at a reduced speed. Thus, it is preferred to utilize a polygon scanner (7a) for said first axis and a galvo scanner (7b) for said second axis. Alternatively, an acousto-optic deflector may be used for either or both of these scanning devices.

Of course, the combination of the galvo scanner and the polygon scanner may be also be introduced in the embodiment shown in Fig. 6. This is shown in Fig. 8.

Moreover, the focusing optics (8) may comprise a focal reducer (8a) and an f-theta-objective (8b) as shown in Figs. 7 and 8. Utilizing a scanning system in combination with standard lenses will yield a spherical focal plane which may not be desirable. In order to achieve a planar focal plane, an f-theta-objective (8b) may be utilized. Moreover, a focal reducer (8a) may be present in order to reduce the area of the illumination pattern generated by the DMD 3 to a predetermined area size acceptable to the scanning devices 7a and 7b.

## Claims

1. A device for re-shaping the cornea (1) of a patient, the device comprising:
a laser source (2);
a digital micromirror device (3), DMD, adapted to emit a pattern of multiple laser beams (4);
a mounting mechanism (6) for fixating the head of a patient;
focusing optics (8) adapted for focusing the pattern of multiple laser beams (4) emitted by the digital micromirror device (3) onto the cornea (1) of the patient;
an image sensor (18) adapted to image the cornea (1) of the patient; and
a controller adapted to control, based on the image detected by the image sensor (18), the laser source (2) and the digital micromirror device (3) so as to generate one or more illumination patterns on the cornea (1) of the patient which allow(s) for re-shaping the cornea (1) of the patient in accordance with a predetermined target shape of the cornea (1) of the patient;
wherein the device further comprises one or more scanning devices (7) for steering the pattern of multiple laser beams (4) emitted by the DMD (3) over the cornea of the patient, and wherein the one or more scanning devices (7) are arranged between the DMD (3) and the focusing optics (8).

2. The device of claim 1, wherein the laser source (2) comprises one or more of a picosecond laser, a femtosecond laser, an excimer laser, preferably both a femtosecond laser and an excimer laser.

3. The device of claim 1 or 2, further comprising a prism (16) or semi-transparent mirror positioned between the laser source (2) and the digital micromirror device (3).

4. The device of any of the previous claims, wherein the focusing optics (8) comprises a lens having a numerical aperture of at least 0.5, more preferably of at least 0.8.

5. The device of any of the previous claims, wherein the device further comprises a beam shaping device, preferably a flat top beam shaper (21), a matrix of laser zone plates (12) or a spatial light modulator.

6. The device of any of the previous claims, further comprising an optical element (12) adapted for creating a Bessel beam shape, wherein the optical element (12) preferably comprises a spatial light modulator, wherein the optical element is preferably incorporated into the digital micromirror device (3).

7. The device of claim 3 or claims 4-6, when dependent on claim 3, further comprising a λ/4 plate (17a) positioned between the prism (16) or the semi-transparent mirror and the digital micromirror device (3).

8. The device of any of the previous claims, further comprising collimating optics (5) for collimating laser light emitted by the laser source (2) onto the digital micromirror device (3).

9. The device of claim 7, wherein the prism (16) or semi-transparent mirror is positioned between the laser source (2) and the digital micromirror device (3) in such a manner that light emitted from the laser source (2), in that order, passes through the prism (16) or semi-transparent mirror, hits the digital micromirror device (3), and again passes through the prism (16) or semi-transparent mirror before selectively illuminating the cornea.

10. The device of any of the previous claims, wherein the one or more scanning devices (7) comprises one or a combination of one or more galvo scanners (7b), one or more polygon scanners (7a), and one or more acousto-optic deflectors, preferably a combination of one galvo scanner and one polygon scanner.

11. The device of any of the previous claims, wherein the focusing optics (8) comprises a focal reducer (8a) and an f-theta-objective (8b), wherein the focal reducer (8a) is preferably arranged between the DMD (3) and the one or more scanning devices (7), wherein the one or more scanning devices (7) are preferably arranged between the focal reducer (8a) and the f-theta-objective (8b).

12. The device of any of the previous claims, wherein the controller is adapted to control, based on the image detected by the image sensor (18), the laser source (2), the one or more scanning devices and the digital micromirror device (3) so as to generate several illumination patterns on the cornea (1) of the patient which allow for re-shaping the cornea (1) of the patient in accordance with a predetermined target shape of the cornea (1) of the patient.

13. The device of any of the previous claims, wherein the controller is adapted to control, based on the image detected by the image sensor (18), the laser source (2) and the digital micromirror device (3), optionally also the one or more scanning devices, so as to generate one or more illumination patterns on the cornea (1) of the patient which allow(s) for creating a flap of the cornea (1) of the patient in accordance with a predetermined shape of the flap of the cornea (1) of the patient, wherein the laser source (2) preferably comprises a femtosecond laser and an excimer laser and wherein the controller is preferably adapted to utilize the femtosecond laser for creating the flap and to utilize the excimer laser for re-shaping the cornea.

14. The device of claim 3, 7 or any of claims 4-6, 8-13 when dependent on claim 3, further comprising a beam splitter (19) positioned between the prism (16) or the semi-transparent mirror and the focusing optics (8) for allowing light emitted from the cornea to pass to the image sensor (18).

15. The device of claim 3, 7, 14 or any of claims 4-6, 8-13 when dependent on claim 3, further comprising a further light source (20) adapted to illuminate the cornea via the prism (16) or semi-transparent mirror and the digital micromirror device (3) and/or via the beam splitter (19).

## Patentansprüche

1. Vorrichtung zur Umformung der Hornhaut (1) eines Patienten, wobei die Vorrichtung aufweist:
eine Laserquelle (2);
eine digitale Mikrospiegelvorrichtung, DMD, (3), die eingerichtet ist, ein Muster aus mehreren Laserstrahlen (4) zu emittieren;
einen Befestigungsmechanismus (6) zum Fixieren des Kopfes eines Patienten;
eine Fokussieroptik (8), die zum Fokussieren des von der digitalen Mikrospiegelvorrichtung (3) emittierten Musters aus mehreren Laserstrahlen (4) auf die Hornhaut (1) des Patienten eingerichtet ist;
einen Bildsensor (18), der eingerichtet ist, die Hornhaut (1) des Patienten abzubilden; und
eine Steuerung, die eingerichtet ist, basierend auf dem vom Bildsensor (18) erfassten Bild die Laserquelle (2) und die digitale Mikrospiegelvorrichtung (3) zu steuern, um ein oder mehrere Beleuchtungsmuster auf der Hornhaut (1) des Patienten zu erzeugen, die eine Umformung der Hornhaut (1) des Patienten gemäß einer vorbestimmten Zielform der Hornhaut (1) des Patienten ermöglichen;
und wobei die eine oder mehreren Scanvorrichtungen (7) zwischen der DMD (3) und der Fokussieroptik (8) angeordnet sind.

2. Vorrichtung nach Anspruch 1, wobei die Laserquelle (2) einen oder mehrere eines Pikosekundenlasers, eines Femtosekundenlasers, eines Excimerlasers aufweist, vorzugsweise sowohl einen Femtosekundenlaser als auch einen Excimerlaser.

3. Vorrichtung nach Anspruch 1 oder 2, die ferner ein Prisma (16) oder einen halbdurchlässigen Spiegel aufweist, der zwischen der Laserquelle (2) und der digitalen Mikrospiegelvorrichtung (3) angeordnet ist.

4. Vorrichtung nach einem der vorhergehenden Ansprüche, wobei die Fokussieroptik (8) eine Linse mit einer numerischen Apertur von mindestens 0,5, vorzugsweise von mindestens 0,8, aufweist.

5. Vorrichtung nach einem der vorhergehenden Ansprüche, wobei die Vorrichtung ferner eine Strahlformungsvorrichtung, vorzugsweise einen Flat-Top-Strahlformer (21), eine Matrix aus Laser-Zonenplatten (12) oder einen räumlichen Lichtmodulator aufweist.

6. Vorrichtung nach einem der vorhergehenden Ansprüche, die ferner ein optisches Element (12) aufweist, das zur Erzeugung einer Bessel-Strahlform eingerichtet ist, wobei das optische Element (12) vorzugsweise einen räumlichen Lichtmodulator aufweist, wobei das optische Element vorzugsweise in die digitale Mikrospiegelvorrichtung (3) eingebaut ist.

7. Vorrichtung nach Anspruch 3 oder nach den Ansprüchen 4 bis 6, sofern von Anspruch 3 abhängig, die ferner ein λ/4-Plättchen (17a) aufweist, das zwischen dem Prisma (16) oder dem halbdurchlässigen Spiegel und der digitalen Mikrospiegelvorrichtung (3) angeordnet ist.

8. Vorrichtung nach einem der vorhergehenden Ansprüche, die ferner eine Kollimationsoptik (5) zum Kollimieren von der Laserquelle (2) emittierten Laserlichts auf die digitale Mikrospiegelvorrichtung (3) aufweist.

9. Vorrichtung nach Anspruch 7, wobei das Prisma (16) oder der halbdurchlässige Spiegel zwischen der Laserquelle (2) und der digitalen Mikrospiegelvorrichtung (3) derart positioniert ist, dass von der Laserquelle (2) emittiertes Licht in dieser Reihenfolge das Prisma (16) oder den halbdurchlässigen Spiegel durchläuft, auf die digitale Mikrospiegelvorrichtung (3) trifft, und erneut das Prisma (16) oder den halbdurchlässigen Spiegel durchläuft, bevor es die Hornhaut selektiv beleuchtet.

10. Vorrichtung nach einem der vorhergehenden Ansprüche, wobei die eine oder die mehreren Scanvorrichtungen (7) einen oder eine Kombination aus einem oder mehreren Galvo-Scannern (7b), einem oder mehreren Polygonscannern (7a) und einem oder mehreren akustooptischen Deflektoren aufweisen, vorzugsweise eine Kombination aus einem Galvo-Scanner und einem Polygonscanner.

11. Vorrichtung nach einem der vorhergehenden Ansprüche, wobei die Fokussieroptik (8) einen Brennweitenreduzierer (8a) und ein f-Theta-Objektiv (8b) aufweist, wobei der Brennweitenreduzierer (8a) vorzugsweise zwischen der DMD (3) und der einen oder den mehreren Scanvorrichtungen (7) angeordnet ist, wobei die eine oder die mehreren Scanvorrichtungen (7) vorzugsweise zwischen dem Brennweitenreduzierer (8a) und dem f-Theta-Objektiv (8b) angeordnet sind.

12. Vorrichtung nach einem der vorhergehenden Ansprüche, wobei die Steuerung eingerichtet ist, basierend auf dem vom Bildsensor (18) erfassten Bild die Laserquelle (2), die eine oder die mehreren Scanvorrichtungen und die digitale Mikrospiegelvorrichtung (3) zu steuern, um mehrere Beleuchtungsmuster auf der Hornhaut (1) des Patienten zu erzeugen, die eine Umformung der Hornhaut (1) des Patienten gemäß einer vorbestimmten Zielform der Hornhaut (1) des Patienten ermöglichen.

13. Vorrichtung nach einem der vorhergehenden Ansprüche, wobei die Steuerung eingerichtet ist, basierend auf dem vom Bildsensor (18) erfassten Bild die Laserquelle (2) und die digitale Mikrospiegelvorrichtung (3), optional auch die eine oder die mehreren Scanvorrichtungen, zu steuern, um ein oder mehrere Beleuchtungsmuster auf der Hornhaut (1) des Patienten zu erzeugen, die das Erzeugen eines Lappens der Hornhaut (1) des Patienten gemäß einer vorbestimmten Form des Lappens der Hornhaut (1) des Patienten ermöglichen, wobei die Laserquelle (2) vorzugsweise einen Femtosekundenlaser und einen Excimerlaser aufweist und wobei die Steuerung vorzugsweise eingerichtet ist, den Femtosekundenlaser zum Erzeugen des Lappens zu nutzen und den Excimerlaser zur Umformung der Hornhaut zu nutzen.

14. Vorrichtung nach Anspruch 3, 7 oder einem der Ansprüche 4 bis 6, 8 bis 13, sofern von Anspruch 3 abhängig, die ferner einen Strahlteiler (19) aufweist, der zwischen dem Prisma (16) oder dem halbdurchlässigen Spiegel und der Fokussieroptik (8) angeordnet ist, um es zu ermöglichen, dass von der Hornhaut emittiertes Licht zum Bildsensor (18) geht.

15. Vorrichtung nach Anspruch 3, 7, 14 oder einem der Ansprüche 4 bis 6, 8 bis 13, sofern von Anspruch 3 abhängig, die ferner eine weitere Lichtquelle (20) aufweist, die eingerichtet ist, die Hornhaut über das Prisma (16) oder den halbdurchlässigen Spiegel und die digitale Mikrospiegelvorrichtung (3) und/oder über den Strahlteiler (19) zu beleuchten.

## Revendications

1. Dispositif de remodelage de la cornée (1) d'un patient, ledit dispositif comprenant :
une source laser (2) ;
un dispositif à micromiroirs numérique, DMD (3), adapté pour émettre un motif de faisceaux laser multiples (4) ;
un mécanisme de fixation (6) destiné à immobiliser la tête d'un patient ;
une optique de focalisation (8) adaptée pour focaliser le motif de faisceaux laser multiples (4) émis par le dispositif à micromiroirs numérique (3) vers la cornée (1) du patient ;
un capteur d'image (18) adapté pour capturer une image de la cornée (1) du patient ; et
un contrôleur adapté pour commander, sur la base de l'image détectée par le capteur d'image (18), la source laser (2) et le dispositif à micromiroirs numérique (3) de manière à générer un ou plusieurs motifs d'éclairage sur la cornée (1) du patient, permettant de remodeler la cornée (1) du patient conformément à une forme cible prédéterminée de la cornée (1) du patient ;
où ledit dispositif comprend en outre un ou plusieurs dispositifs de balayage (7) destinés à diriger le motif de faisceaux laser multiples (4) émis par le DMD (3) sur la cornée du patient, et où le ou les dispositifs de balayage (7) sont disposés entre le DMD (3) et l'optique de focalisation (8).

2. Dispositif selon la revendication 1, où la source laser (2) comprend un laser picoseconde et/ou un laser femtoseconde et/ou un laser excimère, de préférence un laser femtoseconde conjointement avec un laser excimère.

3. Dispositif selon la revendication 1 ou la revendication 2, comprenant en outre un prisme (16) ou un miroir semi-transparent positionné entre la source laser (2) et le dispositif à micromiroirs numérique (3).

4. Dispositif selon l'une des revendications précédentes, où l'optique de focalisation (8) comprend une lentille ayant une ouverture numérique de 0,5 au moins, de préférence de 0,8 au moins.

5. Dispositif selon l'une des revendications précédentes, où le dispositif comprend en outre un dispositif de mise en forme de faisceau, de préférence un dispositif de mise en forme de faisceau à sommet plan (21), une matrice de plaques zonales de laser (12) ou un modulateur spatial de lumière.

6. Dispositif selon l'une des revendications précédentes, comprenant en outre un élément optique (12) adapté pour créer une forme de faisceau de Bessel, où l'élément optique (12) comprend de préférence un modulateur spatial de lumière, et où l'élément optique est de préférence intégré dans le dispositif à micromiroirs numérique (3).

7. Dispositif selon la revendication 3 ou selon les revendications 4 à 6, si dépendantes de la revendication 3, comprenant en outre une plaque λ/4 (17a) positionnée entre le prisme (16) ou le miroir semi-transparent et le dispositif à micromiroirs numérique (3).

8. Dispositif selon l'une des revendications précédentes, comprenant en outre une optique de collimation (5) destinée à collimater la lumière laser émise par la source laser (2) sur le dispositif à micromiroirs numérique (3).

9. Dispositif selon la revendication 7, où le prisme (16) ou le miroir semi-transparent est positionné entre la source laser (2) et le dispositif à micromiroirs numérique (3) de sorte que la lumière émise par la source laser (2), dans l'ordre : traverse le prisme (16) ou le miroir semi-transparent, est incidente sur le dispositif à micromiroirs numérique (3), puis traverse à nouveau le prisme (16) ou le miroir semi-transparent avant d'éclairer de manière sélective la cornée.

10. Dispositif selon l'une des revendications précédentes, où le ou les dispositifs de balayage (7) comprennent un ou plusieurs scanners Galvo (7b), ou un ou plusieurs scanners polygonaux (7a), ou un ou plusieurs déflecteurs acousto-optiques, ou une combinaison de ceux-ci, de préférence une combinaison d'un scanner Galvo et d'un scanner polygonal.

11. Dispositif selon l'une des revendications précédentes, où l'optique de focalisation (8) comprend un réducteur de focale (8a) et un objectif f-thêta (8b), le réducteur de focale (8a) étant de préférence disposé entre le DMD (3) et le ou les dispositifs de balayage (7), le ou les dispositifs de balayage (7) étant de préférence disposés entre le réducteur de focale (8a) et l'objectif f-thêta (8b).

12. Dispositif selon l'une des revendications précédentes, où le contrôleur est adapté pour commander, sur la base de l'image détectée par le capteur d'image (18), la source laser (2), le ou les dispositifs de balayage et le dispositif à micromiroirs numérique (3) de manière à générer plusieurs motifs d'éclairage sur la cornée (1) du patient, permettant de remodeler la cornée (1) du patient conformément à une forme cible prédéterminée de la cornée (1) du patient.

13. Dispositif selon l'une des revendications précédentes, où le contrôleur est adapté pour commander, sur la base de l'image détectée par le capteur d'image (18), la source laser (2) et le dispositif à micromiroirs numérique (3), ainsi que, facultativement, le ou les dispositifs de balayage, de manière à générer un ou plusieurs motifs d'éclairage sur la cornée (1) du patient, permettant de créer un lambeau de la cornée (1) du patient conformément à une forme prédéterminée du lambeau de la cornée (1) du patient, où la source laser (2) comprend de préférence un laser femtoseconde et un laser excimère, et où le contrôleur est de préférence adapté pour utiliser le laser femtoseconde afin de créer le lambeau et pour utiliser le laser excimère afin de remodeler la cornée.

14. Dispositif selon la revendication 3, la revendication 7 ou l'une des revendications 4 à 6, 8 à 13, si dépendantes de la revendication 3, comprenant en outre un séparateur de faisceau (19) positionné entre le prisme (16) ou le miroir semi-transparent et l'optique de focalisation (8) pour permettre à la lumière émise par la cornée d'être transmise au capteur d'image (18).

15. Dispositif selon la revendication 3, la revendication 7, la revendication 14 ou l'une des revendications 4 à 6, 8 à 13, si dépendantes de la revendication 3, comprenant en outre une autre source lumineuse (20) adaptée pour éclairer la cornée via le prisme (16) ou le miroir semi-transparent et le dispositif à micromiroirs numérique (3) et/ou via le séparateur de faisceau (19).
